(19) **Europäisches Patentamt**
**European Patent Office**
**Office européen des brevets**

(11) **EP 1 474 159 B1**

(12) **EUROPEAN PATENT SPECIFICATION**

(45) Date of publication and mention
of the grant of the patent:
**11.04.2007 Bulletin 2007/15**

(21) Application number: **03714737.8**

(22) Date of filing: **10.02.2003**

(51) Int Cl.:
*A61K 36/00* (2006.01)    *A61P 17/00* (2006.01)
*A61P 29/00* (2006.01)    *A61K 9/00* (2006.01)
*A61K 9/70* (2006.01)

(86) International application number:
**PCT/EP2003/001294**

(87) International publication number:
**WO 2003/066073 (14.08.2003 Gazette 2003/33)**

(54) **COMPOSITIONS CONTAINING OAT STRAW AND WILLOWHERB EXTRACT**

ZUSAMMENSETZUNGEN MIT HAFERSTROH UND WEIDEKRAUT-EXTRAKT

COMPOSITIONS CONTENANT DES EXTRAITS DE PAILLE D'AVOINE ET D'EPILOBE

(84) Designated Contracting States:
**AT BE BG CH CY CZ DE DK EE ES FI FR GB GR HU IE IT LI LU MC NL PT SE SI SK TR**

(30) Priority: **08.02.2002 EP 02075535**

(43) Date of publication of application:
**10.11.2004 Bulletin 2004/46**

(73) Proprietor: **JOHNSON & JOHNSON GmbH 40474 Düsseldorf (DE)**

(72) Inventors:
• **LANGE, Rainer**
**53604 Bad Honnef (DE)**
• **VON STETTEN, Otto**
**52072 Aachen (DE)**

(74) Representative: **Weber-Bruls, Dorothée et al Forrester & Boehmert, Pettenkoferstrasse 20-22 80336 München (DE)**

(56) References cited:
**EP-A- 1 078 638          WO-A-00/06116**

**Description**

Background of the Invention

This invention relates to chemical compositions comprising oat straw extract and willowherb extract that can be used to treat or reduce inflammatory reactions on or in the skin. The compositions can be used in topical formulations. The invention further concerns products comprising an applicator, which may be a sheet and a formulation that contains oat straw extract and willowherb extract.

A number of products when applied to the skin may cause inflammatory reactions, which result in phenomena such as irritation, reddening, itching, stinging, eczema, acne and the like. Inflammatory reactions may be caused by certain chemical components that are present in products for topical application or may be caused by exposure of the skin to other external factors such as soils or environmental factors such as air and water pollution or exposure to solar radiation. In these instances the inflammatory reaction causes phenomena such as diaper rash, skin reddening after excessive exposure to the sun, and sunburn.

This is especially the case for products that are applied to sensitive areas of the skin such as the eyes or the region around the eyes, the lips and the area adjacent to the lips, areas of mucous membranes and the like. Furthermore, a number of users have a sensitive skin or have parts of the skin that are particularly sensitive, e.g. people having an atopic skin type. Products that are generally well tolerated may cause adverse effects with such people.

Furthermore, the daily routine of skin cleansing may cause similar phenomena even without intermediation of the products used therein. Regular intense rubbing of the skin and/or regular contact with humidity may cause these phenomena, which may be enhanced by the presence of certain components in the products used thereby. Also exposure to dry and cold air or both can cause these phenomena. Again, sensitive areas of the skin or persons with a sensitive skin will be confronted more intensively with such phenomena.

Hence there is a need for agents that prevent or avoid inflammatory reactions on or in the skin. These agents could be used as additives in topical formulations, or special topical formulations containing these agents could be developed which would be specifically aimed for use prior to, during of after exposure to formulations or other factors that cause inflammatory reactions on or in the skin. Providing such agents and formulations containing these agents are objects of this invention.

One particular way to apply topical formulations is via suitable applicators such as wipes. Recently, wipe products have become an important product category that has found a wide variety of applications for adults and babies. Examples include face or body cleansing wipes, wipes for skin treatment or skin conditioning, wipes for administering active ingredients such as sunscreen wipes. These products are typically manufactured by impregnating sheets made of non-woven fabric with a suitable lotion.

Over the years, wipe products have undergone tremendous changes and many innovations have been introduced such as improvements in the fabric, in the impregnating liquid (or lotion) as well as in product presentation.

Wipes are often loaded with formulations that cleanse and/or moisturize the skin. During use, the wipes may contact sensitive areas, such as mucous membranes or the eyes. This may cause inflammatory reactions, in particular when using the wipes on the sensitive areas or when applying the wipes on users with a sensitive skin type. The same in fact applies also to other types of applicator products.

Hence there is a need for applicator products and in particular for wipe products that contain formulations that do not initiate inflammatory reactions. There is a further need for agents that prevent or reduce the inflammatory reactions caused by certain products present on the wipe or by the wipe material itself. Providing such products and agents are objects of this invention.

These objects are attained by applying the compositions or formulations of this invention to applicators or in particular to wipes, whereby such applicator or wipe products prevent or reduce inflammatory reactions on or in the skin.

A particularly important product attribute of wipes is softness. Softness of the wipe material on the one hand and softness of the skin after usage of the wipe on the other, are important consumer benefits. This in particular is the case for applications on babies.

Initially, wet wipe products were made of traditional non-woven materials based on paper making technology (pulp based products). These products were well accepted but deficient in softness of the fabric material. The introduction of the 'spunlace' non-woven technology offered products that, compared to traditional paper based products, were superior in terms of softness.

Softness of non-woven fabrics can also be improved by adding softeners to either the finished product or to the fibers used as raw materials. This in particular has been applied in dry facial tissue products and toilet paper, where softness has been significantly improved via the addition of 'fabric softeners'. Most of these softeners are silicon-based compounds or derivatives thereof.

Hence there is a need to provide wipe products that are superior in terms of softness while at the same time reduce or treat inflammatory reactions on or in the skin, softness relating as well to the wipe product itself, as to the skin after having been treated with the wipe product. Providing such wipes is an additional goal of this invention.

Summary of the Invention

The present invention concerns chemical compositions comprising oat straw extract and willowherb extract.

The invention is also concerned with formulations for topical use comprising a composition as defined herein and further ingredients. The topical formulations can be for dermatological use but are in particular for cosmetic use. In a particular aspect the topical formulations are for cleansing.

The compositions or formulations of the invention may contain additional active ingredients such as other plant extracts. In particular embodiments, the compositions or formulations additionally contain Aloe vera extract and/or chamomile extract.

In another aspect, the invention provides a composition as defined herein for treating or reducing inflammatory reactions on or in the skin.

In a further aspect the invention provides a cosmetic a composition as defined herein, or a topical formulation as defined herein, for reducing or treating inflammatory reactions on or in the skin.

Still another aspect of this invention comprises a cosmetic for the improvement of the external appearance of an individual.

Particular topical formulations are lotions for use on applicators, which are sheets, and more in particular wipes. Hence the present invention concerns products comprising an applicator, which is a porous or absorbent sheet and a formulation that contains oat straw extract and willowherb extract. Preferably the formulation is liquid and is coated onto or impregnated into said applicator or said sheet.

In a preferred embodiment, the compositions or formulations are liquid. They contain glyceryl monooleate and a $C_{8-20}$ alkyl glucoside. In a more preferred embodiment, the compositions or formulations additionally contain Aloe vera extract and/or chamomile extract.

The compositions or formulations of the invention can be a water-based, in particular they can be aqueous solutions. The compositions or formulations preferably are emulsion-based in which the emulsion can be water-in-oil or oil-in-water or can be of more complex nature such as water-in-oil-in-water. Preferred are oil-in-water emulsions, more preferably oil-in-water emulsions prepared according to the phase inversion technique.

In a further aspect the invention concerns the use of a formulation as defined herein to treat or reduce reactions due to inflammatory reactions. In still a further aspect these formulations additionally improve skin softness. Or alternatively, the invention concerns a method to reduce or treat adverse reactions due to inflammatory reactions, which comprises applying a product as defined herein to the skin.

Detailed Description of the Invention

Whenever used in this description and claims, any percentage is weight by weight (w/w).

The oat straw and willowherb extracts for use in the compositions and formulations of this invention are obtained by art known extraction procedures from natural sources of oat straw (Avena sativa) and willowherb (Epilobium angustifolium, also referred to as fireweed plant). The extracts may be aqueous, with various concentrations of dry material; or they may be in dry form. The extracts in particular are aqueous and may further contain components selected from preservatives, in particular preservatives commonly used in cosmetic formulations, suitable solvents such as mono- and/or polyols, e.g. ethanol, ethylene glycol, propylene glycol, glycerine and the like, and other components such as preservatives, stabilizing agents and the like.

Preferred oat straw and willowherb extracts are aqueous and contain from about 0.5 to about 10 % of dry material, preferably from about 1 to about 5 % more preferably from about 2 to about 3 % of dry material.

A particularly preferred oat straw extract is the product called 'Extrapone Oats™' which is commercially available from the company Dragoco, Germany. This oat straw extract is aqueous and contains from about 1 % to about 5 % of dry oat straw extract (Avena sativa).

A particularly preferred willowherb extract is the product called 'Canadian Willowherb Extract™' which is commercially available from the company Fytokem (Atrium Biotechnologies), Canada. This willowherb extract is aqueous and contains from about 1 % to about 5 % of dry willowherb extract (Epilobium angustifolium).

The percentages in the previous paragraphs are w/w, i.e. weight of the dry extract to the total weight of the extract solution.

The w/w ratio of both extracts in the compositions of the invention may vary but in particular is in the range of 10 : 1 to 1 : 10, more in particular in the range of the range of 5 : 1 to 1 : 5, or more specifically in the range of the range of 3 : 1 to 1 : 3. Of interest are compositions wherein the ratio of the amounts of oat straw extract to willowherb extract is in the range of 5 : 1 to 1 : 1. Preferred compositions are those wherein the w/w ratio of the amounts of oat straw extract to willowherb extract is about 1 : 1 or is about 5 : 1. As used herein, the w/w ratios refer to weights of dry extract. The extracts can be used in solvents, if desired containing other materials, and the w/w ratio is then to be recalculated in accordance with the quantity of solvent or other materials present in the extract.

The oat straw extract, when in solution at the w/w concentrations of about 1 % to about 5 % of the commercially available extracts, can be used in the formulations for topical application at concentrations from 0.001 % to 10 % and preferably from 0.002 % to 5%, most preferably between 0.005 % and 3%. The willowherb extract, when in solution at the w/w concentrations of about 1 % to about 5 %, can be used in the formulations for topical application at a concentration between from 0.001 % to 10 % and preferably from 0.002 % to 5 %, most preferably between 0.005 % and 3%.

In particular, the oat straw extract, expressed as w/w concentrations of dry material, can be used in the formulations for topical application at concentrations from 0.00001 % to 0.5 % and preferably from 0.00002 % to 0.25 %, most preferably between 0.00005 % and 0.15 %. Further in particular, the oat straw extract, expressed as w/w concentrations of dry material, can be used in the formulations for topical application at concentrations from 0.001 % to 0.05 % and preferably from 0.002 % to 0.01 %, most preferably between 0.005 % and 0.025 %.

Additionally in particular, the willowherb extract, expressed as w/w concentrations of dry material, can be used in the formulations for topical application at concentrations from 0.00001 % to 0.5 % and preferably from 0.00002 % to 0.25 %, most preferably between 0.00005 % and 0.15 %. Further in particular, the willowherb extract, expressed as w/w concentrations of dry material, can be used in the formulations for topical application at concentrations 0.0001% to 0.05 % and preferably from 0.00015 to 0.01 %, most preferably between 0.001 % and 0.005 %. As used in this and the previous paragraph, the perecentages of oat straw extract or willowherb relate to dry material and where the extract is used in solution, the above percentages evidently need to be recalculated taking into consideration the concentration of the dry extract in the solution.

The compositions of the invention are prepared by mixing the extracts, optionally in the presence of a suitable solvent. If desired, other components may be added. Alternatively, the oat straw and willowherb extract may be mixed with the other components and the thus prepared mixtures may be combined to the desired composition.

The compositions may also take the form of concentrates, which subsequently are transformed into the desired composition by addition of solvent and/or other components.

Preferably, the compositions are aqueous. They may additionally contain further ingredients, including the ingredients mentioned herein for use in the topical formulations of the invention. In particular they may contain further active ingredients.

In a preferred embodiment the compositions of this invention further contain Aloe vera extract obtained from the extraction of Aloe Barbadensis leaf juice. A particularly preferred Aloe vera extract is commercially available under the trade name 'Extrapone Aloe Vera™, from Dragoco, Germany. This extract contains from about 0.1 % to about 1 % of Aloe vera.

The w/w ratio of the Aloe vera extract to the willowherb extract in the compositions of the invention may vary but in particular is in the range of 10 : 1 to 1 : 10, more in particular in the range of the range of 5 : 1 to 1 : 5, or more specifically in the range of the range of 3 : 1 to 1 : 3. Preferably said ratio is about 1 : 1. As used herein, these w/w ratios refer to dry extract and should be recalculated if the extracts are used in solvents.

In another embodiment the compositions of this invention further contain Chamomile extract (Chamomilla Recutita). A particularly preferred Chamomile extract is commercially available under the trade name 'Extrapone Chamomile™ from Dragoco, Germany.

The w/w ratio of the Chamomile extract to the willowherb extract in the compositions of the invention may vary but in particular is in the range of 10 : 1 to 1 : 10, more in particular in the range of the range of 5 : 1 to 1 : 5, or more specifically in the range of the range of 3 : 1 to 1 : 3. Preferably said ratio is about 1 : 1. Here also, the w/w ratios refer to dry extracts.

The compositions of this invention contain specific components aimed at improving the tactile sensation of the compositions and of the formulations derived there from. In particular agents increasing the softness of the composition and the softness of the skin after application can be added. In the particular case of compositions, or the formulations derived there from, for use in or on applicators (wipes), agents are added to improve softness of the applicator (wipe).

This agent is glyceryl monooleate. The amount of the latter in the compositions of the invention will be such that, after addition of appropriate other components, the concentration of glyceryl monooleate in the end formulation is in the range from 0.01 to 2 %, in particular from 0.015 to 1 %, preferably from 0.0175 to 0.5 %, more preferably in the range from 0.0175 to 0.335 %, or from 0.02 to 0.5 % still more preferably from 0.08 to 0.2 %.

The compositions of the invention also contain a fatty alcohol glucoside, viz. a $C_{8-20}$ alkyl glucoside, more in particular a $C_{8-16}$ alkyl glucoside, preferably coco-glucoside. These compounds are also referred to as alkyl glycosides or coco-glycoside.

The amount of said glucoside in the compositions of the invention will be such that, after addition of appropriate other components, the concentration of said glucoside in the end formulation in particular is in the range from 0.01 to 2 %, in particular from 0.015 to 1 %, preferably from 0.0175 to 0.5 %, more preferably in the range from 0.0175 to 0.335 %, or from 0.02 to 0.5 % still more preferably from 0.08 to 0.2 %.

As used herein $C_{8-20}$ alkyl or $C_{8-16}$ alkyl refers to straight or branch chained hydrocarbon radicals, saturated or unsaturated, having from about 8 to about 20 or from about 8 to about 16 carbon atoms, including mixtures thereof. $C_{8-20}$ alkyl or $C_{8-16}$ alkyl in particular is derived from fatty alcohols. Examples of $C_{8-16}$ alkyl are capryl, 2-ethylhexyl, caprinyl, lauryl, isotridecyl, myristyl, palmoleyl, cetyl, and the like. $C_{8-20}$ alkyl comprises these radicals as well as stearyl, isostearyl, oleyl, linolenyl, linolyl, and the like.

The ratio of the amount of glyceryl monooleate to the fatty alcohol glucoside in the compositions and formulations derived thereof is in the range from 2 : 1 to 1 : 2, preferably in the range from 1.5 : 1 to 1 : 1.5, most preferably said ratio is about 1 : 1.

A particularly suited combination is that which is sold under the trademark 'Lamesoft™', in particular 'Lamesoft PO65', a mixture of 20 to 40 % of glyceryl monooleate, 20 to 40 % of coco glucoside and water. This 'Lamesoft' product is added to the formulations in an amount in the range from 0.1 to 1 %, preferably from 0.1 to 0.5 %, more preferably from 0.25 to 0.5 %.

The compositions according to the present invention can be prepared by mixing the extracts and optional extracts or ingredients. The extracts may be dry or in solution, and after mixing, further solvent may be added or solvent, if present, may be evaporated.

This invention further relates to topical formulations containing a composition as defined herein. Topical compositions comprise dermatological formulations (or topical pharmaceutical formulations), as well as cosmetical formulations. The topical formulations of the invention may come in different forms. They may be solid, semi-solid or liquid, for example they can take the form of solutions, emulsions, pastes, powders, granulates, gels, lotions including lotions for wipes, after-sun lotions and the like. The formulations of the invention may be those typically used in dermatology or cosmetology such as bath additives, creams, ointments, gels, lotions, and the like or formulations for cleansing such as shampoos, soaps, cleansing lotions, and the like. The formulations may be, for example, in the form of solutions, gels, lotions, creams, oil-in-water, water- in-oil or multiple emulsions, foaming products or in liposome form.

Said topical formulations may further contain other ingredients or additives suited for external topical administration, in particular dermatologically or cosmetically acceptable ingredients or additives, including other active ingredients. Examples of further ingredients or additives are surfactants, emulsifiers, consistency factors, conditioners, emollients, skin caring ingredients, moisturizers, softeners, thickeners, lubricants, fillers, binding agents, anti-oxidants, preservatives, active ingredients, in particular dermatologically active ingredients, dyes, opacifying agents fragrances and the like. Active ingredients as mentioned herein comprise, for example, anti-inflammatories, anti-bacterials, anti-fungals and the like agents. Any such further active ingredients should be suited for topical applications.

Oils that can be added are of natural or synthetic origin, e.g. vegetable oils or mineral oils or of silicone oils. Examples of oils are natural oils, e.g. almond oil, soybean oil, wheat germ oil, avocado oil, jojoba oil, linseed oil, sesame oil, walnut oil, sunflower oil, olive oil, etc., mineral and paraffin oil and synthetic oils comprising mono-, di-, triglycerides as well as mixtures thereof.

Emollients that can be added comprise lipids like lanolin, lanolin alcohols, lanolin acids, polyethoxylated or acylated lanolin or lanolin derivatives, lecithin and lecithin derivatives, fatty alcohols, either linear or branched with chain lengths between C6 and C40, and their esters with organic acids, e.g. carbonic acids or polyacids containing between 2 and 30 C atoms, branched, aromatic or linear including hydroxy or amino acids, fatty acids and fatty acid esters with alcohols or poly alcohols containing between 2 and 40 C atoms, branched, aromatic or linear, sterols found in the unsaponifiable fraction of e.g. avocado oil, almond oil, soybean oil, etc. like soy phytosterol, β-sitosterol, β-sitosteryl laurate, β-sitosteryl stearate, etc. Natural and synthetic waxes, e.g. bees wax, purcelline, shea butter, cocoa butter, ceresin, ozokerit, vaseline, micro wax, carnauba wax, candelilla wax and alike, substituted cyclohexanes like di-n-octylcyclohexane, Guerbet carbonates; e.g. bis-2-octyl dodecylcarbonate, dialkyl ethers like di-n-octyl ether, etc.

The formulations may also contain film-forming substances like chitosan and derivatives thereof, derivatives of polyacrylic acid, polyvinyl pyrrolidone and its derivatives, etc.

The formulations may further contain active ingredients such as anti-microbials, e.g. anti-bacterials and anti-fungals, anti-inflammatory agents, anti-irritants, anti-itching agents, anti-stinging agents, moisturizing agents, skin caring ingredients, plant extracts, vitamins, and the like. Examples of such ingredients comprise complexes of PVP and hydrogen peroxide, anti-inflammatories as, plant extracts, bisabolol, panthenol, tocopherol, actives for anti-stinging, anti-dandruffs, for anti-ageing e.g. retinoids such as retinal, retinol or retinoic acid or derivatives thereof such as retinyl palmitate, retinyl acetate etc., carbohydrates such as melibiose, ethanolamines such as dimethylaminoethanol and its salts, etc. Other suitable actives are e.g. Medicago officinalis, Actinidia chinensis, allantoin, Aloe barbadensis, Anona cherimolia, Anthemis nobilis, Arachis hypogaea, Arnica montana, Avena sativa, beta-carotene, bisabolol, Borago officinalis, butylene glycol, Calendula officinalis, Camellia sinensis, camphor, Candida bombicola, capryloyl glycine, Carica papaya, Centaurea cyanus, cetylpyridinium chloride, Chamomilla recutita, Chenopodium quinoa, Chinchona succirubra, Chondrus crispus, Citrus aurantium dulcis, Citrus grandis, Citrus limonum, Cocos nucifera, Coffea arabica, copper peptides such as copper tripeptide-1, Crataegus monogina, Cucumis melo, dichlorophenyl imidazoldioxolan, Enteromorpha compressa, Equisetum arvense, ethanolamines such as dimethylaminoethanol and their salts, ethoxydiglycol, ethyl panthenol, farnesol, ferulic acid, Fragaria chiloensis, Gentiana lutea, Ginkgo biloba, glyceryl laurate, Glycyrrhiza glabra, Hamamelis virginiana, heliotropine, hydrogenated palm glycerides, citrates, hydrolyzed castor oil, hydrolyzed wheat protein, Hypericum perforatum, Iris florentina, Juniperus communis, lactis proteinum, lactose, Lawsonia inermis, linalool, Linum usitatissimum, lysine, magnesium aspartate, magnifera indica, Malva sylvestris, mannitol, mel, Melaleuca alternifolia, Mentha piperita, menthol, menthyl lactate, Mimosa tenuiflora, Nymphaea alba, olaflur, Oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, Persea gratissima, petrolatum, potassium aspartate, potassium sorbate, propylene glycol, Prunus amygdalus dulcis, Prunus armeniaca, Prunus persica, Ricinus communis, Rosa canina, Rosmarinus officinalis, Rubus idaeus, salicylic acid, Sambucus nigra, sarcosine, Serenoa serrulata, Simmondsia chinensis, sodium carboxymethyl betaglucan, sodium cocoyl amino acids, sodium hyaluronate, sodium palmitoyl proline, stearoxytrimethylsilane, sulfurized TEA-ricinoleate, talcum, thymus vulgaris, Tilia cordata, tocopherol, tocopheryl acetate, trideceth-9, Triticum vulgare, tyrosine, undecylenoyl glycine, urea, Vaccinium myrtillus, valine, zinc oxide, zinc sulfate and the like.

The formulations according to the present invention can be prepared by adding the appropriate ingredients to a composition of the invention, or vice versa by adding the composition to an appropriate cosmetic or dermatological formulation base. It is also possible to mix all the ingredients individually, i.e. without making a separate composition as defined herein.

The topical formulations of this invention can be applied on the skin by means of appropriate applicators, i.e. applicators in the form of sheets such as wipes.

Embodiments of the present invention therefore are formulations for use on applicators. Hence in a further embodiment there are provided products comprising an applicator and a formulation as defined herein, hereinafter referred to as 'products of the invention'.

The applicators may be made of a variety of materials, which are structured such that they are capable of holding and/or absorbing a lotion. The materials of which the applicators are made therefore is porous or absorbent in nature. The materials in particular are polymeric and may be both from natural and non-natural origin.

The applicator can be resilient or non-resilient.

The applicators can be of different size and take a variety of forms. One or more of the outer sides of the applicator may be made of different materials having different properties. For example one side may be soft while another side is rougher. The latter side can be abrasive, it can be used for rubbing or scouring.

The applicators can be made of materials that are capable of holding, adsorbing or absorbing a lotion. The applicator material is structured such that it is porous or absorbent in nature. The latter can be due to the chemical structure of the applicator materials or their physical arrangement or both. Examples of particular physical arrangements are porous structures, or cellular or microcellular structures.

The applicators can be made of one type of material or from different materials that can be arranged in different manners along the applicator. Small portions of one or more materials of different or equal size may be incorporated into a matrix of the same or another material. Or the applicators can be multilayered such as a stack of layers or concentric layers or they can be of one type of material. Applicator parts, either or not made of different materials can be linked together by gluing, sewing, stitching or any other technique known in the art.

Applicators are sheets, more preferably wipes. Formulations for wipes sometimes are referred to in the art as 'lotions'.

The sheet of absorbent or porous material for use in the products of this invention can take the form of a tissue, a wipe, towel, towelette, and the like. The material may be flushable. As used herein, by 'flushable' is meant that the material will pass through at least 3.3 m of waste pipe in two toilet flushes. The material may also be biodegradable.

Sheet materials that can be used can be mono- or multi-layered, woven or non-woven. They can be made of one or of several materials. Particularly preferred are non-woven materials that have a web structure of fibrous or filamentous nature, in which the fibers or filaments are distributed randomly or with a certain degree of orientation, the former being obtainable by air-laying or by certain wet-laying processes, the latter by other wet-laying or by carding processes. The fibers or filaments can be natural, for example wood pulp, wool cotton, linen and the like, or synthetic, for example polyvinyls, polyesters, polyolefins, polyamides and the like. Typically they have a weight per square meter in the range of 10 to 80 g/m$^2$, in particular of 20 to 70 g/m$^2$. Particular materials are of the non-woven type. Based on the raw material that has been used, two different types of products can be distinguished.

A first type of carriers is paper-based. The raw materials for these carriers are made almost exclusively of cellulose-based fibers or filaments from plant cellular sources (pulp). In a number of wipe applications, such as baby wipes, wipes for cleansing, wet paper towels and the like, high wet strength or firmness of the non-woven web is a desirable attribute. This can be achieved by the addition of binding materials, e.g. the so-called wet strength resins. In some cases additives are added in order to increase the softness of the end product.

In a second type the web is made mainly of staple fibers, e.g. based on cotton, wool, linen and the like.

Commercial products are made of cellulose fibers, synthetic fibers or mixtures of both. Polyester and polypropylene are known as suitable polymers for the preparation of synthetic fibers. Also in these products binders can be used to increase the firmness of the non-woven fabric.

Webs of increased strength can be obtained by using the so-called spunlace or hydro-entanglement technique. In this technique the individual fibers are twisted together so that an acceptable strength or firmness is obtained without using binding materials. The advantage of the latter technique is the excellent softness of the non-woven material.

Non-woven materials that are made of mixtures of pulp and staple fiber are also known. Such materials are available with binding materials, in particular those mentioned above, or without binding materials. In the latter instance the non-woven is preferably made by the spunlace or hydro-entanglement procedure. The carrier material preferably is made of cellulose pulp with a small amount of binding material. The amount of binder in the carrier material is in the range of 5 to 20% (w/w). In particularly preferred embodiments, the non-woven fabric is prepared by the water-entanglement procedure and does not contain binding material.

The absorbing ability of the sheet material is of particular importance with regard to the applications envisaged by the present invention. During production the lotion should be taken up quickly by the carrier. In certain embodiments of this invention the wipes will be packed in a stack of a plurality of wipes. In this instance the absorbing ability of the non-woven fabric should be such that a chromatographic effect (sinking down of the lotion) in the stack is avoided during storage. On the other hand it should be guaranteed that during the usage of the wipe the lotion is delivered evenly to the skin. The absorbing capacity of the carrier material is determined essentially by three different parameters: the surface weight of the carrier material, the nature of the raw material used in the manufacture and the manufacturing process used.

For the applications according to the invention the carrier materials typically have a surface weight from 10 $g/m^2$ to 80 $g/m^2$, preferably from 30 to 70 $g/m^2$ and more preferably from 40 to 60 $g/m^2$. The selection of the raw material of which the non-woven carrier is made depends on the manufacturing procedure. Typically in the manufacture of non-woven carriers by the hydro-entanglement process, use is made of mixtures of cellulose fibers and synthetic fibers. The relative quantity of synthetic fibers in the non-woven fabric is from 0 to 100% and preferably is between 10 and 70%, more preferably in the range of 30 to 50% (all percentages being w/w).

In particular, the formulations in the products of the invention are liquid. They can be water-based formulations, in particular they can take the form of aqueous solutions. The liquids preferably are emulsion-based. These liquid compositions, which also are referred to as 'lotions', preferably are of aqueous nature.

The emulsions can be oil-in-water or water-in-oil emulsions, or be of more complex nature such as water-in-oil-in-water. Preferred are oil-in-water emulsions, more in particular oil-in-water emulsions prepared according to the phase inversion technique.

The formulations for use in or on the applicators (wipes) contain softeners such as glyceryl monooleate and a fatty alcohol glucoside, both being as described hereinabove and being present in concentrations and ratios also as described above.

The amount of the formulations on the sheets or wipes will be in the range from about 100% to about 400%, preferably from about 200% to about 400%, expressed as the weight of the composition relative to the weight of the sheet in dry condition.

Preferred compositions or formulations of the invention are those based on emulsions prepared by the so-called phase inversion technique. This is particularly the case for formulations in or on wipes. The phase inversion technique is described in more detail by F. Förster, F. Schambil, and H. Tesmann in Int. J. Cos. Sci. 1990 (12) 217. Particular PTT emulsions that can be used in the compositions of this invention are described for example in WO-00/51427, in WO-00/71676 and in WO-00/04230. The PIT compositions can be prepared starting from materials and using methods known in the art and in particular described in the references mentioned herein.

Preferred are PIT emulsions that are finely dispersed, i.e. having a small droplet size and have low viscosity.

The products of the invention can be made by coating the said composition onto or impregnating it into the sheet material. The term coating also comprises techniques such as printing and spraying of the composition on the sheet. In a preferred embodiment, wipes are impregnated with a lotion prepared according to the PIT procedure.

The compositions or formulations for use in the products of the invention are prepared by conventional methods. In a particular embodiment a concentrate is made which subsequently is diluted with a suitable aqueous medium to obtain the composition that is applied to the sheet. The concentrate may be formulated as an emulsion, in particular as an oil-in-water emulsion, more in particular as an oil-in-water emulsion prepared according to the PIT technique. In that instance the concentrate will contain appropriate emulsifiers, in particular those mentioned herein.

The concentration of the plants extracts and other actives in the concentrate may be from about 2.5 to about 10 times, preferably from about 5 to about 7.5 times higher than the concentration thereof in the end formulation.

Hence the invention concerns a process for preparing product of the invention as defined herein, said process comprising contacting a suitable applicator with a formulation as described herein. In particular said process comprises impregnating a wipe with a liquid formulation as described herein, more in particular impregnating or spraying a wipe with a liquid or lotion. The latter preferably is a PIT formulation as described herein.

In a particular execution, the sheet material is cut into strips the transversal size of which being similar to the size of the sheet, in particular the tissue or wipe. Subsequently the carrier strips are folded according to methods generally known and applied in the art. The thus folded strips are moistened with a liquid formulation as defined herein, in particular with a PIT composition, said moistening preferably comprising spraying or dripping. Or the fabric strips can first be moistened and subsequently be folded.

The strips can also be impregnated with the formulation by immersing in or running the strip through a bath containing the composition. They can also be sprayed or printed with the formulation.

In a further step, the strips are cut so that the desired size of the sheets, in particular of the wipes, is obtained. The thus obtained sheets (or wipes) can be packed individually or can be stacked in a determined number, e.g. a number between 10 and 30, preferably between 15 and 25, most preferably about 20, or a number between 50 and 100, preferably between 60 and 80, most preferably about 72, and the stack then packed in a suitable package, for example a plastic wrap, box and the like.

The products of the invention can take the form of baby or adult wipes and can be used in a wide range of applications as personal care products, comprising, for example, baby cleansing wipes, face or body cleansing wipes, wipes for skin treatment or skin conditioning such as for example skin moisturization, insect repellent wipes, sunscreen wipes, and the like.

As used herein applying or application to the skin comprises any action of contacting the product to the skin e.g. by rubbing across the skin, bathing, dabbing, wetting and the like.

The products of the invention, when applied to the skin, cause a sufficient or an effective amount of actives to be released and thereby administered to the skin. Additionally the present products allow an even distribution of the active ingredients onto the skin.

The topical formulations that are subject of the present invention are particularly useful to treat or reduce the effects of inflammation. The latter in particular comprise the effects mentioned in the background section. The formulations of the present invention further can be used as soothing agents.

A particular aspect of this invention comprises the fact that oat straw extract and willowherb extract act synergistically. For example these combinations show synergistic effects in their capability to reduce or treat the effects of inflammation.

According to another aspect, the invention concerns the use of a combination of oat straw extract with willowherb extract for the preparation of a composition designed to reduce or treat the effects of inflammation.

The invention further provides a method for the cosmetic treatment of the effects of inflammation, which consists in applying an effective amount of the compositions or formulations as described herein.

The products of the invention have superior properties in terms of softness, feel and cleansing. Softness is the tactile sensation perceived when the consumer holds the product, rubs it across the skin, or crumples it with the hand. Increased softness is present when using glyceryl monooleate and a fatty alcohol glucoside. The products show increased softness of the sheet material, more specifically they show increased softness of the non-woven fabric. Additionally, they result in an improvement of the softness of the skin in that it has a softer feel or an improved sensorial softness after application of the wipe. They further result in increased skin smoothness and skin elasticity.

Examples

As used in these examples 'Nipaguard IPF' is a trademark for a preservative containing 10 % of iodopropyl butylcarbamate (IPBC).

'Ceteareth-20' is ethoxylated cetostearyl alcohol having 20 ethoxy units;
'Ceteareth-12' is ethoxylated cetostearyl alcohol having 12 ethoxy units.

Example 1

Preparation of a concentrate

| | |
|---|---|
| Glyceryl stearate/ Ceteareth 20/ Ceteareth 12 / Cetearyl alcohol/ cetyl palmitate mixture | 13.00 % |
| Mineral Oil | 15.00 % |
| Phenoxyethanol | 0.83 % |
| Nipaguard IPF™ | 0.50 % |
| Fragrance | 1.0 % |

(continued)

| | |
|---|---|
| Aqua | 69.67 % |

The above ingredients are mixed and subsequently slowly warmed above the PTT temperature. This is determined by measuring the electrical conductivity of the mixture. Subsequently the mixture is allowed to cool slowly to room temperature. The thus obtained PIT formulation has the appearance of a clear solution with low viscosity.

Preparation of a final lotion (impregnant)

Extrapone Oats™ and Extrapone Aloe Vera™ are available from Dragoco, Germany and are trade names of oat straw extract containing 1 - 5 % of Avena sativa, and Aloe vera extract containing 0.1-1 % of Aloe Barbadensis juice extract. Canadian
Willowherb Extract™ is available from Fytokem (Atrium Biotechnologies), Canada and contains 1 - 5 % of Epilobium Angustifolium.

| | Formulation 1 | Formulation 2 | Comparative Formulation 3 |
|---|---|---|---|
| Aqua | 77.036 % | 77.636 % | 77.886 % |
| Tetrasodium EDTA (chelating agent) | 0.20 % | 0.20 % | 0.20 % |
| Phenoxyethanol | 0.834 % | 0.834 % | 0.834 % |
| Cetylpyridinium chloride (CPC) | 0.05 % | 0.05 % | 0.05 % |
| Lamesoft PO65™ | 0.25 % | 0.25 % | - |
| Concentrate as described above | 20.00 % | 20.00 % | 20.0 % |
| Extrapone Oats™ | 0.50 % | 0.50 % | 0.50 % |
| Canadian Willowherb Extract™ | 0.10 % | 0.50 % | 0.50 % |
| Extrapone Aloe Vera™ | 1.0 % | - | - |
| Citric Acid Monohydrate | 0.03 % | 0.03 % | 0.03 % |

To the required amount of water there is added a premix of tetrasodium EDTA, phenoxyethanol, cetylpyridinium chloride and Lamesoft PO65™, while stirring (no Lamesoft PO65™ is added in comparative Formulation 3). Stirring is continued until a homogeneous mixture is obtained. Subsequently, the concentrate as described above is added. Stirring is continued until the mixture turns homogeneous. Then, under further stirring, the oat straw, willowherb and Aloe vera extracts are added, followed by the addition of citric acid monohydrate for pH adjustment.

Comparative Example 2

| | Formulation 4 | Formulation 5 |
|---|---|---|
| Aqua | 96.02 % | 96.62 % |
| Tetrasodium EDTA (chelating agent) | 0.20 % | 0.20 % |
| Nipaguard IPF™ | 0.1 % | 0.1 % |
| Phenoxyethanol | 1.0 % | 0.167 % |
| Cetylpyridinium chloride (CPC) | 0.05 % | 0.05 % |
| Propylene glycol | 0.8 % | 0.8 % |
| Extrapone Oats™ | 0.5 % | 0.5 % |
| Canadian Willowherb Extract™ | 0.1 % | 0.5 % |
| Extrapone Aloe Vera™ | 1.0 % | - |

9

(continued)

|  | Formulation 4 | Formulation 5 |
|---|---|---|
| Polysorbate-20 | 0.2 % | 0.2 % |
| Citric Acid Monohydrate | 0.03 % | 0.03 % |

A first premix is prepared by mixing the required amounts of tetrasodium EDTA, Nipaguard IPF™, phenoxyethanol, cetylpyridinium chloride and propylene glycol until a homogeneous mixture is obtained. This first premix is added to the water under continuous stirring. When 100% batch homogeneity of the mixture is achieved, a second premix, obtained by mixing the oat straw and willowherb extraxts with polysorbate, is added to the main batch (in formulation 4 the Aloe Vera is also added to the second premix). When a homogeneous mixture is achieved, citric acid monohydrate is added stepwise to adjust pH.

Example 3

Dry hydro-entangled carrier material made of fabric having a surface weight of 50 g/m$^2$ is cut into strips. The strips are sprayed in the conventional manner with the liquid as prepared in example 2. Liquid addition was set at 6 g per wipe. Subsequently the strips are folded and cut.

Example 4

The forearm of human volunteers was challenged with sodium laurylsulfate solution under occlusion for 4 hours to provoke an inflammatory reaction shown by redness of the test area. The test compounds and control water were applied to the site. Redness was measured using a chromameter daily for 5 days. Redness reduction (Rred) was calculated according to the following equation :

$$\text{Rred (\%)} = (\text{R after challenge} - \text{R day}_x) \times 100 / (\text{R after challenge} - \text{R at start})$$

The extracts were incorporated in water. The concentrations used were :

| | |
|---|---|
| Oat Straw | 1.0% in water |
| Willowherb | 1.0% in water |
| Oat Straw / Willowherb | 1.0% in water (0.5% / 0.5%) |

Results :

The combination of oat straw and willowherb is more active than the individual components alone at the same total concentration of active material.

| | 2 days | 3 days | 4 days | 5 days |
|---|---|---|---|---|
| Oat Straw | 61 % | 90 % | 69 % | 100 % |
| Willowherb | 39 % | 66 % | 83 % | 100 % |
| Oat Straw / Willowherb | 45 % | 90 % | >100 % | >100 % |

>100% means that the skin redness was less than initial redness (i.e. skin redness prior to the challenge with sodium lauryl sulfate).

**Claims**

1. A product comprising a porous or absorbent sheet and a topical formulation comprising glyceryl monooleate and a $C_{8-20}$ alkyl glucoside and a chemical composition comprising oat straw extract and willowherb extract and further ingredients.

2. A composition according to claim 1, additionally containing Aloe vera extract and/or chamomile extract.

3. The product according to claim 1 or 2 wherein the composition is a liquid that is coated onto or impregnated into said sheet.

4. The product according to claims 1 or 2 wherein the composition is an oil-in-water emulsion.

5. The product according to claim 4 wherein the oil-in-water emulsion is prepared according to the phase inversion technique.

6. The product according to claim 1 wherein said further ingredients comprise surfactants, emulsifiers, consistency factors, conditioners, emollients, skin caring ingredients, moisturizers, softeners, thickeners, lubricants, fillers, binding agents, anti-oxidants, preservatives, dyes, opacifying agents, fragrances, anti-inflammatories, anti-bacterials or anti-fungals.

**Patentansprüche**

1. Produkt, umfassend ein poröses oder absorptionsfähiges Flächengebilde und eine topische Formulierung, umfassend Glycerylmonooleat und ein $C_{8-20}$-Alkylglucosid und eine chemische Zusammensetzung, umfassend Haferstrohextrakt und Weidenröschenextrakt und weitere Bestandteile.

2. Zusammensetzung gemäß Anspruch 1, zusätzlich enthaltend Aloe-Vera-Extrakt und/oder Kamillenextrakt.

3. Das Produkt nach Anspruch 1 oder 2, **dadurch gekennzeichnet, daß** die Zusammensetzung eine Flüssigkeit ist, mit der besagtes Flächengebilde beschichtet oder imprägniert wird.

4. Das Produkt nach Anspruch 1 oder 2, **dadurch gekennzeichnet, daß** die Zusammensetzung eine Öl-in-Wasser-Emulsion ist.

5. Das Produkt nach Anspruch 4, **dadurch gekennzeichnet, daß** die Öl-in-Wasser-Emulsion nach dem Phaseninversionsverfahren hergestellt ist.

6. Das Produkt nach Anspruch 1, **dadurch gekennzeichnet, daß** besagte weitere Bestandteile grenzflächenaktive Stoffe, Emulgatoren, Konsistenzfaktoren, Konditionierer, Emollientien, Hautpflegebestandteile, Feuchthaltemittel, Weichmacher, Verdicker, Schmierstoffe, Füllstoffe, Bindemittel, Antioxidationsmittel, Konservierungsstoffe, Farbstoffe, Trübungsmittel, Duftstoffe, entzündungshemmende Stoffe, antibakterielle Stoffe oder antifungale Stoffe umfassen.

**Revendications**

1. Produit comprenant une feuille poreuse ou absorbante et une formulation topique comprenant du monooléate de glycéryle et un glucoside alkylique en $C_8$-$C_{20}$ et une composition chimique comprenant un extrait de paille d'avoine et un extrait d'épilobe et d'autres ingrédients.

2. Composition selon la revendication 1, contenant en outre un extrait d'aloe vera et/ou un extrait de camomille.

3. Produit selon la revendication 1 ou 2 dans lequel la composition est un liquide qui est déposé sur ou imprégné dans ladite feuille.

4. Produit selon la revendication 1 ou 2 dans lequel la composition est une émulsion huile dans l'eau.

5. Produit selon la revendication 4 dans lequel l'émulsion huile dans l'eau est préparée selon la technique d'inversion de phase.

6. Produit selon la revendication 1 dans lequel lesdits autres ingrédients comprennent des agents tensioactifs, des émulsifiants, des facteurs de consistance, des conditionneurs, des émollients, des ingrédients de soin pour la peau, des hydratants, des adoucissants, des épaississants, des lubrifiants, des charges, des agents de liaison, des antioxydants, des conservateurs, des teintures, des agents opacifiants, des parfums, des anti-inflammatoires, des antibactériens ou des antifongiques.